(19) Europäisches Patentamt
European Patent Office
Office européen des brevets

(11) Veröffentlichungsnummer: **0 478 721 B1**

(12) # EUROPÄISCHE PATENTSCHRIFT

(45) Veröffentlichungstag der Patentschrift: **06.09.95**

(51) Int. Cl.$^6$: **C07C 62/38**, C07C 51/64, A61K 31/19, C07B 57/00

(21) Anmeldenummer: **91905069.0**

(22) Anmeldetag: **19.03.91**

(86) Internationale Anmeldenummer:
**PCT/CH91/00064**

(87) Internationale Veröffentlichungsnummer:
**WO 91/14671 (03.10.91 91/23)**

(54) **NEUE BENZOCYCLOALKENCARBONSÄURE UND VERFAHREN ZU IHRER HERSTELLUNG.**

(30) Priorität: **27.03.90 CH 1010/90**

(43) Veröffentlichungstag der Anmeldung:
**08.04.92 Patentblatt 92/15**

(45) Bekanntmachung des Hinweises auf die Patenterteilung:
**06.09.95 Patentblatt 95/36**

(84) Benannte Vertragsstaaten:
**AT BE CH DE DK ES FR GB GR IT LI LU NL SE**

(56) Entgegenhaltungen:
**EP-A- 0 132 566**

**Ullmanns Encyclopädie der technischen Chemie, 4. Auflage, Band 17, veröffentlicht von Verlag Chemie (Weinheim, New York), Seiten 451-454,**

(73) Patentinhaber: **CIBA-GEIGY AG**
**Klybeckstrasse 141**
**CH-4002 Basel (CH)**

(72) Erfinder: **VON SPRECHER, Andreas**
**Nelkenweg 1**
**CH-4104 Oberwil (CH)**
Erfinder: **WETTER, Hansjürg**
**Vogesenstrasse 22**
**CH-4106 Therwil (CH)**

**EP 0 478 721 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die neue (S)-4-(2-Brombenzoyl)-5-hydroxy-benzocyclobuten-1-carbonsäure, d. h. die Verbindung der Formel

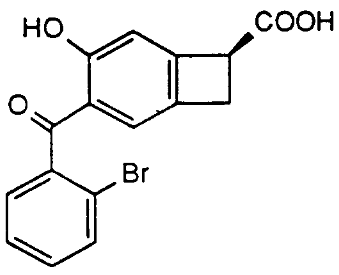

(I),

in freier Form oder in Salzform, die Verwendung dieser Verbindung, ein Verfahren zur Herstellung dieser Verbindung und pharmazeutische Präparate, enthaltend die Verbindung I in freier Form oder in Form eines pharmazeutisch verwendbaren Salzes.

Die Verbindung I kann als, insbesondere pharmazeutisch verwendbares, Salz vorliegen. Entsprechende Salze sind Salze mit Basen. Geeignete Salze mit Basen sind z. B. Metallsalze, wie Alkalimetallsalze, z. B. Natrium- oder Kaliumsalze, oder wie Erdalkalimetallsalze, z. B. Calcium- oder Magnesiumsalze, oder Salze mit Ammoniak, mit einem organischen Amin, wie Morpholin, Thiomorpholin, Piperidin, Pyrrolidin oder einem gegebenenfalls C-hydroxylierten aliphatischen Amin, z. B. einem Mono-, Di- oder Triniederalkylamin, wie Methyl-, Ethyl- oder Diethylamin, einem Mono-, Di- oder Trihydroxyniederalkylamin, wie Ethanol-, Diethanol- oder Triethanolamin, Tris(hydroxymethyl)methylamin oder 2-Hydroxy-tert.-butylamin, einem N-(Hydroxyniederalkyl)-N,N-diniederalkyl-amin, wie 2-(Dimethylamino)ethanol, oder einem N-(Polyhydroxyniederalkyl)-N-niederalkyl-amin, wie D-Glucamin, oder mit einer quaternären Ammoniumbase, wie einem quaternären aliphatischen Ammoniumhydroxid, z.B. Tetrabutylammoniumhydroxid. Umfasst sind ferner für pharmazeutische Verwendungen nicht geeignete Salze, die z. B. für die Isolierung bzw. Reinigung der Verbindung I, in freier Form oder in Form eines pharmazeutisch verwendbaren Salzes, eingesetzt werden.

Die racemische 4-(2-Brombenzoyl)-5-hydroxy-benzocyclobuten-1-carbonsäure, d. h. das 1:1 -Enantiomerengemisch von (R)- und (S)-4-(2-Brombenzoyl)-5-hydroxy-benzocyclobuten-1-carbonsäure, ist bekannt und wird, beispielsweise in der europäischen Patentanmeldung Nr. 0 132 566, als antinociceptiver und antiinflammatorischer Arzneimittelwirkstoff mit die Prostaglandinsynthese hemmenden Eigenschaften vorgeschlagen. Die neue (S)-4-(2-Brombenzoyl)-5-hydroxy-benzocyclobuten-1-carbonsäure, in freier Form oder in pharmazeutisch verwendbarer Salzform, weist ebenfalls wertvolle pharmakologische Eigenschaften auf, z. B. antinociceptive, antiinflammatorische und die Prostaglandinsynthese hemmende Wirkungen, welche in ihrem Ausmass mit den Wirkungen des bekannten Racemats vergleichbar sind. So zeigt die (S)-4-(2-Brombenzoyl)-5-hydroxy-benzocyclobuten-1-carbonsäure z. B. entsprechende erwünschte Wirkungen in dem Writhing-Testmodell (Induktion durch Phenyl-p-benzochinon) an der Maus [nach J. Pharmacol. exp. Therap. 125, 237 (1959)], wobei die $ED_{50}$ 5 mg/kg p. o. beträgt, sowie in dem Adjuvans-Arthritis-Test an der Ratte, in welchem eine $ED_{40}$ von 0,5 mg/kg p. o. ermittelt wird. Zusätzlich weist dem bekannten Racemat gegenüber das erfindungsgemäss bereitgestellte (S)-Enantiomere jedoch überraschende therapeutische Vorteile auf. Insbesondere wird das (S)-Enantiomere in geringerem Ausmass im Fettgewebe akkumuliert. So ergeben Vergleichsversuche mit $^{14}C$-markierter (S)-4-(2-Brombenzoyl)-5-hydroxy-benzocyclobuten-1-carbonsäure und $^{14}C$-markierter racemischer 4-(2-Brombenzoyl)-5-hydroxy-benzocyclobuten-1-carbonsäure an der Ratte, dass bei täglicher Applikation von je 5 mg/kg p.o. über einen Zeitraum von 10 Tagen bei Verabreichung des (S)-Enantiomeren radioaktives Material in niedrigerer Konzentration in das Fettgewebe eingelagert wird als bei Verabreichung des Racemats. Dies ist vor allem im Hinblick auf eine chronische Verabreichung des Wirkstoffs als erheblicher therapeutischer Vorteil des (S)-Enantiomeren gegenüber dem Racemat zu werten.

Dementsprechend kann die Verbindung I, in freier Form oder in pharmazeutisch verwendbarer Salzform, z.B. als Wirkstoff in Antinociceptiva, Antiinflammatorika und Prostaglandinsythesehemmern verwendet werden, welche z.B. zur Behandlung von Entzündungen, wie entzündlichen Erkrankungen des rheumatischen Formenkreises, z. B. der chronischen Arthritis, eingesetzt werden. Ein Erfindungsgegenstand ist somit die Verwendung der Verbindung I, in freier Form oder in pharmazeutisch verwendbarer Salzform, zur Herstellung von entsprechenden Arzneimitteln und zur therapeutischen Behandlung von Entzündungen, wie entzündlichen Erkrankungen des rheumatischen Formenkreises, z. B. der chronischen Arthritis. Bei der

Herstellung der Arzneimittel ist auch die gewerbsmässige Herrichtung der Wirksubstanzen eingeschlossen.

In der europäischen Patentanmeldung Nr. 0 132 566 ist angegeben, dass die racemische 4-(2-Brombenzoyl)-5-hydroxy-benzocyclobuten-1-carbonsäure nach bekannten Methoden in die Enantiomeren aufgetrennt werden kann, beispielsweise durch Umsetzung "mit einer mit der racemischen Säure Salze bildenden optisch aktiven Base und Trennung der auf diese Weise erhaltenen Salze, z. B. auf Grund ihrer verschiedenen Löslichkeiten, in die Diastereomeren, aus denen die Antipoden durch Einwirkung geeigneter Mittel freigesetzt werden" können. Über die Art der zu verwendenden optisch aktiven Base fehlen in der europäischen Patentanmeldung Nr. 0 132 566 jedoch nähere Angaben. Verwendet man z. B. Chinin als optisch aktive Base und arbeitet man nach üblichen Verfahren der fraktionierten Kristallisation, erhält man nur mässige Ausbeuten an (S)-4-(2-Brombenzoyl)-5-hydroxy-benzocyclobuten-1-carbonsäure (siehe Vergleichsbeispiel 1). Auch durch Erhöhung der Kristallisationsstufenzahl kann die Ausbeute an dem gewünschten (S)-Enantiomeren nicht verbessert werden (siehe Vergleichsbeispiel 2).

Es wurde nun übertaschenderweise festgestellt, dass man (S)-4-(2-Brombenzoyl)-5-hydroxy-benzocyclobuten-1-carbonsäure von hoher bis vollständiger, beispielsweise mindestens 85 %-iger, d. h. 85 %-iger bis 100 %-iger, wie etwa 90 %-iger bis etwa 99 %-iger, beispielsweise mindestens 92 %-iger, optischer Reinheit in Ausbeuten von über 90 % der Theorie erhält, wenn man racemische 4-(2-Brombenzoyl)-5-hydroxy-benzocyclobuten-1-carbonsäure in einem alkoholischen Lösungsmittel etwa 6 bis etwa 48 Stunden bei erhöhter Temperatur mit mindestens der äquimolaren Menge Chinin umsetzt, das als unmittelbares Umsetzungsprodukt kristallin ausgefallene Chininsalz der (S)-4-(2-Brombenzoyl)-5-hydroxy-benzocyclobuten-1-carbonsäure aus dem Reaktionsgemisch abtrennt und aus diesem Salz durch Säurebehandiung in üblicher Weise die (S)-4-(2-Brombenzoyl)-5-hydroxy-benzocyclobuten-1-carbonsäure freisetzt.

Ein weiterer Gegenstand der Erfindung ist daher ein neues Verfahren zur Herstellung der Verbindung I, in freier Form oder in Salzform, dadurch gekennzeichnet, dass man racemische 4-(2-Brombenzoyl)-5-hydroxy-benzocyclobuten-1-carbonsäure in einem alkoholischen Lösungsmittel 6 bis 48 Stunden bei erhöhter Temperatur mit mindestens der äquimolaren Menge Chinin umsetzt, das als unmittelbares Umsetzungsprodukt kristallin ausgefallene Chininsalz der (S)-4-(2-Brombenzoyl)-5-hydroxy-benzocyclobuten-1-carbonsäure aus dem Reaktionsgemisch abtrennt und aus diesem Salz durch Säurebehandlung in üblicher Weise die (S)-4-(2-Brombenzoyl)-5-hydroxy-benzocyclobuten-1-carbonsäure freisetzt und diese freie Säure gewünschtenfalls in ein Salz überführt.

Als alkoholische Lösungsmittel kommen insbesondere Niederalkanole, d. h. $C_1$-$C_7$-Alkanole, wie $C_1$-$C_4$-Alkanole, beispielsweise Methanol, Ethanol, Propanol, Isopropanol oder Butanol, insbesondere Ethanol, in Betracht. Die Menge des alkoholischen Lösungsmittels ist nicht kritisch; es wird jedoch mindestens die zur vollständigen Auflösung der Komponenten erforderliche Alkoholmenge benötigt. Pro g der racemischen 4-(2-Brombenzoyl)-5-hydroxy-benzocyclobuten-1-carbonsäure werden beispielsweise 8 bis 50 ml, vorzugsweise 20 bis 30 ml, des alkoholischen Lösungsmittels veranschlagt.

Die Reaktionszeit beträgt etwa 6 bis etwa 48 Stunden in den meisten Fällen vorteilhaft 12 bis 24 Stunden.

Die Reaktionstemperatur ist nicht kritisch, sofern sie gegenüber der Raumtemperatur deutlich erhöht ist. Vorteilhaft arbeitet man beispielsweise in einem Temperaturbereich von 50° bis 120° C, insbesondere zwischen 60° und 90° C, vorzugsweise bei der Siedetemperatur des verwendeten alkoholischen Lösungsmittels.

Die Chininmenge ist nicht kritisch, sofern mindestens die äquimolare Menge Chinin eingesetzt wird. Als günstig erweist sich beispielsweise die 1,0-fache bis 1,5-fache, vorzugsweise die 1,05-fache bis 1,3-fache, insbesondere die 1,15-fache bis 1,25-fache, molare Menge Chinin.

Die Abtrennung des verfahrensgemäss als unmittelbares Umsetzungsprodukt kristallin ausgefallenen Chininsalzes der (S)-4-(2-Brombenzoyl)-5-hydroxy-benzocyclobuten 1-carbonsäure aus dem Reaktionsgemisch erfolgt in üblicher Weise, beispielsweise durch Filtration, Saugfiltration (Abnutschen) oder Zentrifugation.

Die Freisetzung der (S)-4-(2-Brombenzoyl)-5-hydroxy-benzocyclobuten-1-carbonsäure durch Säurebehandlung ihres verfahrensgemäss erhaltenen Chininsalzes erfolgt in üblicher Weise, beispielsweise durch Behandlung mit einer Mineralsäure, wie einer Halogenwasserstoffsäure, z. B. Salzsäure. Dabei verwendet man mindestens die äquimolare, d. h. mindestens die 1-fache, beispielsweise die 2-fache bis 10-fache, insbesondere die 2-fache bis 5-fache, molare Säuremenge und arbeitet vorteilhaft in einem Zweiphasensystem, gebildet aus Wasser und einem mit Wasser nicht-mischbaren oder nur teilweise mischbaren organischen Lösungsmittel, wie einem aliphatischen Alkohol, wie einem $C_4$-$C_7$-Alkanol, z. B. Butanol, Isobutanol, sek.-Butanol tert.-Butanol oder einem Pentanol, Hexanol oder Heptanol, einem niederen Fettsäureester, wie einem $C_2$-$C_7$-Alkansäure-$C_1$-$C_4$-alkylester, z. B. Essigsäureethylester, einem aromatischen oder araliphatischen Kohlenwasserstoff, z. B. Benzol oder einem Alkylderivat davon, wie Toluol oder Xylol, einem

halogenierten aliphatischen Kohlenwasserstoff, wie einem Halogen-$C_1$-$C_4$-alkan, z. B. Di- oder Trichlormethan, oder einem aliphatischen Ether, wie einem Di-$C_1$-$C_4$-alkylether, z. B. tert.-Butylmethylether.

In einer bevorzugten Ausführungsform des erfindungsgemässen Verfahrens erwärmt man eine Lösung von racemischer 4-(2-Brombenzoyl)-5-hydroxybenzocyclobuten-1-carbonsäure und der 1,0-fachen bis 1,5-fachen molaren Menge, d. h. pro kg Säure 0,94 bis 1,41 kg, Chinin in der 8-fachen bis 50-fachen Volumenmenge, d.h. pro kg Säure 8 bis 50 l, eines $C_1$-$C_4$-Alkanols 6 bis 48 Stunden auf 60° bis 90° C, trennt das als unmittelbares Umsetzungsprodukt kristallin ausgefallene Chininsalz der (S)-4-(2-Brombenzoyl)-5-hydroxy-benzocyclobuten-1-carbonsäure in üblicher Weise aus dem Reaktionsgemisch ab und setzt die (S)-4-(2-Brombenzoyl)-5-hydroxy-benzocyclobuten-1-carbonsäure in üblicher Weise durch Säurebehandlung ihres verfahrensgemäss erhaltenen Chininsalzes frei.

In einer besonders bevorzugten Ausführungsform des erfindungsgemässen Verfahrens erwärmt man eine Lösung von racemischer 4-(2-Brombenzoyl)-5-hydroxy-benzocyclobuten-1-carbonsäure und der 1,05-fachen bis 1,3-fachen molaren Menge, d. h. pro kg Säure 0,99 bis 1,22 kg, Chinin in der 20-fachen bis 30-fachen Volumenmenge, d. h. pro kg Säure 20 bis etwa 30 l, Ethanol 12 bis 24 Stunden auf die Siedetemperatur des Ethanols, trennt das als unmittelbares Umsetzungsprodukt kristallin ausgefallene Chininsalz der (S)-4-(2-Brombenzoyl)-5-hydroxy-benzocyclobuten-1-carbonsäure in üblicher Weise aus dem Reaktionsgemisch ab und setzt die (S)-4-(2-Brombenzoyl)-5-hydroxy-benzocyclobuten-1-carbonsäure durch Behandlung ihres verfahrensgemäss erhaltenen Chininsalzes mit der 2-fachen bis 5-fachen molaren Menge Salzsäure in einem Zweiphasensystem, gebildet aus Wasser und tert.-Butylmethylether, frei.

Die verfahrensgemäss erhältliche freie (S)-4-(2-Brombenzoyl)-5-hydroxy-benzocyclobuten-1-carbonsäure kann in an sich bekannter Weise in eines ihrer Salze überführt werden, z. B. durch Umsetzung einer Lösung der freien Säure in einem geeigneten Lösungsmittel oder Lösungsmittelgemisch mit einer Base, wie einem Alkalimetallhydroxid, einem Metallcarbonat oder -hydrogencarbonat, Ammoniak oder einer anderen der vorstehend genannten salzbildenden Basen, oder mit einem geeigneten Ionenaustauscherteagens.

Je nach Verfahrensweise bzw. Reaktionsbedingungen kann die Verbindung I in freier Form oder in Salzform erhalten werden. Infolge der engen Beziehung zwischen der Verbindung I in freier Form und in Form ihrer Salze sind im Vorausgegangenen und nachfolgend unter der freien Verbindung I bzw. ihren Salzen sinn- und zweckgemäss gegebenenfalls auch die entsprechenden Salze bzw. die freie Verbindung I zu verstehen.

Die Verbindung I einschliesslich ihrer Salze kann auch in Form von Hydraten erhalten werden und/oder andere, z. B. zur Kristallisation verwendete, Lösungsmittel einschliessen.

Die Verbindung I und ihre pharmazeutisch verwendbaren Salze können, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Antinociceptiva, Antiinflammatorika und Prostaglandinsynthesehemmer verwendet werden.

Die Erfindung betrifft daher gleichfalls pharmazeutische Präparate, die die Verbindung I in freier Form oder in Form eines pharmazeutisch verwendbaren Salzes als Wirkstoff enthalten, sowie ein Verfahren zu ihrer Herstellung. Bei diesen pharmazeutischen Präparaten handelt es sich um solche zur enteralen, wie oralen, ferner rektalen, oder parenteralen Verabreichung an Warmblüter, wobei der pharmakologische Wirkstoff allein oder zusammen mit üblichen pharmazeutischen Hilfsstoffen enthalten ist. Die pharmazeurischen Präparate enthalten z.B. von 0,1 % bis 100 %, vorzugsweise von 1 % bis 50 %, des Wirkstoffs. Pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren, hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepraparate und/oder Calciumphosphate, z.B. Tricalciumphosphat oder Calciumhydrogenphosphat, ferner Bindemittel, wie Stärkekleister, unter Venwendung z.B. von Mais-, Weizen-, Reis- oder Kartoffelstärke, Gelatine, Tragantgummi, Methylcellulose und/oder Polyvinylpyrrolidon, und, wenn erwünscht, Sprengmittel, wie die obengenannten Stärken, ferner Carboxymethylstärke, quervernetztes Polyvinylpyrrolidon, Agar oder Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaftresistenten Übertügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine sowie weiche, geschlossene Kapseln aus Gelatine und einem Weichmacher, wie Glycerin oder Sorbitol. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole und höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole und Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet, oder wässrige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffes kann von verschiedenen Faktoren, wie Applikationsweise, Warmblüter-Spezies, Alter und/oder individuellem Zustand, abhängen. Im Normalfall ist für einen 75 kg schweren Patienten bei oraler Applikation eine ungefähre Tagesdosis von 10 mg bis 250 mg zu veranschlagen.

Die nachfolgenden Beispiele illustrieren die oben beschriebene Erfindung; sie sollen jedoch diese in ihrem Umfang in keiner Weise einschränken. Temperaturen sind in Grad Celsius angegeben.

Ausführungsbeispiel:

a) 3,47 g (10 mmol) racemische 4-(2-Brombenzoyl)-5-hydroxy-benzocyclobuten-1-carbonsäure werden in 40 ml absolutem Ethanol gelöst. Man erwärmt auf 70° und gibt eine Lösung von 3,89 g (12 mmol) Chinin in 40 ml absolutem Ethanol zu. Die Temperatur des Gemisches sinkt auf 58°. Das Reaktionsgemisch wird unter Rühren zum Rückfluss (Innentemperatur: 77°) erhitzt. Nach etwa 30 Minuten beginnt an der Gefasswand die Abscheidung von Kristallen. Man erhitzt das Gemisch weitere 20,5 Stunden unter Rühren zum Rückfluss, lässt es auf Raumtemperatur abkühlen, saugt es ab, wäscht den Filterrückstand zweimal mit je 5 ml absolutem Ethanol und trocknet ihn bis zur Gewichtskonstanz. Man erhält so 6,31 g des Chininsalzes der (S)-4-(2-Brombenzoyl)-5-hydroxy-benzocyclobuten-1-carbonsäure [Ausbeute: 94,1 % der Theorie: (S):(R)-Verhältnis gemäss HPLC-Analyse: ≧96:4].

b) Eine Suspension von 61,5 g (91,7 mmol) des gemäss Stufe a) erhaltenen Chininsalzes der (S)-4-(2-Brombenzoyl)-5-hydroxy-benzocyclobuten-1-carbonsäure in 200 ml tert.-Butylmethylether wird unter Rühren bei 0° mit 100 ml 2 N-Salzsäure versetzt. Die organische Phase wird abgetrennt und die wässrige Phase mit 20 ml tert.-Butylmethylether ausgeschüttelt. Die organischen Phasen werden vereinigt, mit Wasser neutral gewaschen, über Natriumsulfat getrocknet und unter vermindertem Druck zur Trockne eingedampft. Der Rückstand besteht aus 35,2 g eines hochviskosen gelblichen Öles, welches noch etwas Lösungsmittel enthält und aus Hexan zur Kristallisation gebracht wird. Man erhält so 31,4 g (S)-4-(2-Brombenzoyl)-5-hydroxy-benzocyclobuten-1-carbonsäure [Smp.: 104 bis 105°; $[\alpha]_{20}^{D}$ : -24,8° (1 % in $CHCl_3$); (S):(R)-Verhältnis gemäss HPLC-Analyse: ≧ 96:4]. Die Gesamtausbeute über beide Stufen beträgt 93,1 % der Theorie.

Vergleichsbeispiel 1: 17,35 g (50,1 mmol) racemische 4-(2-Brombenzoyl)-5-hydroxybenzocyclobuten-1-carbonsäure werden in 250 ml absolutem Ethanol suspendiert. Man erwärmt die Suspension auf 40°, gibt 8,5 g (26,2 mmol) Chinin zu und erhitzt das Gemisch zum Rückfluss. Bereits bei einer Temperatur von 60 bis 65° beginnt das Chininsalz der 4-(2-Brombenzoyl)-5-hydroxy-benzocyclobuten-1-carbonsäure auszufallen. Man rührt das Reaktionsgemisch 5 Minuten unter Rückfluss, lässt es dann innerhalb von 1 Stunde auf Raumtemperatur abkühlen, saugt die weisse Suspension ab, wäscht den Filterkuchen zweimal mit je 30 ml

absolutem Ethanol und saugt ihn trocken. Man erhält so das Chininsalz der 4-(2-Brombenzoyl)-5-hydroxy-benzocyclobuten-1-carbonsäure (Kristallisationsstufe 1). Dieses Salz wird in 200 ml Essigsäureethylester gelöst und die Lösung mit 100 ml 2 N-Salzsäure ausgeschüttelt, zweimal mit je 50 ml Wasser gewaschen, über Magnesiumsulfat getrocknet und unter vermindertem Druck eingedampft. Die so erhaltene 4-(2-Brombenzoyl)-5-hydroxybenzocyclobuten-1-carbonsäure (Kristallisationsstufe 1) wird in 100 ml absolutem Ethanol gelöst, die Lösung mit 8,1 g (25 mmol) Chinin versetzt und das Reaktionsgemisch zum Rückfluss erhitzt und 5 Minuten unter Rückfluss gerührt. Man lässt das Reaktionsgemisch innerhalb von 1 Stunde auf Raumtemperatur abkühlen, saugt es ab, wäscht den Filterkuchen zweimal mit je 20 ml absolutem Ethanol und saugt ihn trocken. Man erhält so das Chininsalz der (S)-4-(2-Brombenzoyl)-5-hydroxy-benzocyclobuten-1-carbonsäure (Kristallisationsstufe 2). Dieses Salz wird in 200 ml Essigsäureethylester gelöst und die Lösung mit 100 ml 2 N-Salzsäure ausgeschüttelt, zweimal mit je 50 ml Wasser gewaschen, über Magnesiumsulfat getrocknet und unter vermindertem Druck eingedampft. Die so erhaltene rohe (S)-4-(2-Brombenzoyl)-5-hydroxy-benzocyclobuten-1-carbonsäure (Kristallisationsstufe 2) wird in Diethylether gelöst und die Lösung bis zur beginnenden Trübung mit Petrolether versetzt. Man rührt das Gemisch einige Zeit, saugt es ab und trocknet den Filterkuchen über Nacht unter vermindertem Druck bei 40°. Man erhält so 4,7 g reine (S)-4-(2-Brombenzoyl)-5-hydroxy-benzocyclobuten-1-carbonsäure [Smp.: 103 bis 104°; $[\alpha]_{20}^{D}$ : -24,1° ± 1,1°; (S):(R)-Verhältnis gemäss HPLC-Analyse: ≧ 99:1; Gesamtausbeute: 27,1 % der Theorie].

Vergleichsbeispiel 2: 34,7 g (100 mmol) racemische 4-(2-Brombenzoyl)-5-hydroxybenzocyclobuten-1-carbonsäure werden in 800 ml absolutem Ethanol gelöst. Man erwärmt die Lösung auf 40°, gibt 32,4 g (100 mmol) Chinin zu und erhitzt das Reaktionsgemisch zum Rückfluss. Bereits bei einer Temperatur von 70° beginnt das Chininsalz der 4-(2-Brombenzoyl)-5-hydroxy-benzocyclobuten-1-carbonsäure auszufallen. Man rührt das Reaktionsgemisch 5 Minuten unter Rückfluss, lässt es dann innerhalb von 1 Stunde auf 35° abkühlen, saugt die weisse Suspension ab, wäscht den Filterkuchen zweimal mit je 100 ml absolutem Ethanol und saugt ihn trocken. Man erhält so das Chininsalz der 4-(2-Brombenzoyl)-5-hydroxy-benzocyclobuten-1-carbonsäure (Kristallisationsstufe 1). Dieses Salz wird in 200 ml Essigsäureethylester gelöst und die Lösung mit 200 ml 2 N-Salzsäure ausgeschüttelt, zweimal mit je 50 ml Wasser gewaschen, über Magnesiumsulfat getrocknet und unter vermindertem Druck eingedampft. Man erhält so 24 g 4-(2-Brombenzoyl)-5-hydroxy-benzocyclobuten-1-carbonsäure (Kristallisationsstufe 1). Diese Säure wird in 800 ml absolutem Ethanol gelöst, die Lösung mit 22,4 g (69 mmol) Chinin versetzt und das Reaktionsgemisch zum Rückfluss erhitzt und 5 Minuten unter Rückfluss gerührt. Man lässt das Reaktionsgemisch innerhalb von 1 Stunde auf 35° abkühlen, saugt es ab, wäscht den Filterkuchen zweimal mit je 60 ml absolutem Ethanol und saugt ihn trocken. Man erhält so das Chininsalz der 4-(2-Brombenzoyl)-5-hydroxybenzocyclobuten-1-carbonsäure (Kristallisationsstufe 2). Dieses Salz wird in 200 ml Essigsäureethylester gelöst und die Lösung mit 135 ml 2 N-Salzsäure ausgeschüttelt, zweimal mit je 50 ml Wasser gewaschen, über Magnesiumsulfat getrocknet und unter vermindertem Druck eingedampft. Man erhält so 17 g 4-(2-Brombenzoyl)-5-hydroxybenzocyclobuten-1-carbonsäure (Kristallisationsstufe 2). Diese Säure wird in 800 ml absolutem Ethanol gelöst, die Lösung mit 15,9 g (49 mmol) Chinin versetzt und das Reaktionsgemisch zum Rückfluss erhitzt und 5 Minuten unter Rückfluss gerührt. Man lässt das Reaktionsgemisch innerhalb von 1 Stunde auf 35° abkühlen, saugt es ab, wäscht den Filterkuchen zweimal mit je 60 ml absolutem Ethanol und saugt ihn trocken. Man erhält so das Chininsalz der 4-(2-Brombenzoyl)-5-hydroxy-benzocyclobuten-1-carbon saure (Kristallisationsstufe 3). Dieses Salz wird in 200 ml Essigsäureethylester gelöst und die Lösung mit 98 ml 2 N-Salzsäure ausgeschüttelt, zweimal mit je 40 ml Wasser gewaschen, über Magnesiumsulfat getrocknet und unter vermindertem Druck eingedampft. Man erhält so 14 g 4-(2-Brombenzoyl)-5-hydroxy-benzocyclobuten-1-carbonsäure (Kristallisationsstufe 3). Diese Säure wird in 800 ml absolutem Ethanol gelöst, die Lösung mit 13,1 g (40 mmol) Chinin versetzt und das Reaktionsgemisch zum Rückfluss erhitzt und 5 Minuten unter Rückfluss gerührt. Man lässt das Reaktionsgemisch innerhalb von 1 Stunde auf 35° abkühlen, saugt es ab, wäscht den Filterkuchen zweimal mit je 60 ml absolutem Ethanol und saugt ihn trocken. Man erhält so das Chininsalz der (S)-4-(2-Brombenzoyl)-5-hydroxy-benzocyclobuten-1-carbonsäure (Kristallisationsstufe 4). Dieses Salz wird in 200 ml Essigsäureethylester gelöst und die Lösung mit 81 ml 2 N-Salzsäure ausgeschüttelt, zweimal mit je 40 ml Wasser gewaschen, über Magnesiumsulfat getrocknet und unter vermindertem Druck eingedampft. Man erhält so 10,5 g rohe (S)-4-(2-Brombenzoyl)-5-hydroxy-benzocyclobuten-1-carbonsäure (Kristallisationsstufe 4). Diese Säure wird in Diethylether gelöst und die Lösung bis zur beginnenden Trübung mit Petrolether versetzt. Man rührt das Gemisch einige Zeit, saugt es ab und trocknet den Filterkuchen über Nacht unter vermindertem Druck bei 40°. Man erhält so 8,8 g reine (S)-4-(2-Brombenzoyl)-5-hydroxy-benzocyclobuten-1-carbonsäure [Smp.: 103 bis 104°; $[\alpha]_{20}^{D}$ : -26,1° ± 1,0°; (S):(R)-Verhältnis gemäss HPLC-Analyse: ≧ 99,5:0,5; Gesamtausbeute: 25,4 % der Theorie].

Präparatebeispiel 1: Tabletten, als Wirkstoff enthaltend je 50 mg der (S)-4-(2-Brombenzoyl)-5-hydroxy-benzocyclobuten-1-carbonsäure oder eines Salzes, z. B. des Natriumsalzes, davon, können wie folgt

hergestellt werden:

Zusammensetzung (für 10000 Tabletten):

| | |
|---|---|
| Wirkstoff | 500,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g der Kartoffelstärke vermischt und die Mischung mit einer ethanolischen Lösung der Gelatine befeuchtet und durch ein Sieb granuliert. Nach dem Trocknen mischt man den Rest der Kartoffelstärke, das Magnesiumstearat, den Talk und das Siliciumdioxid zu und presst das Gemisch zu Tabletten von je 145 mg Gewicht und 50 mg Wirkstoffgehalt, die gewünschtenfalls mit Teilkerben zur feineren Anpassung der Dosierung versehen sein können.

Präparatebeispiel 2: Lacktabletten, als Wirkstoff enthaltend je 100 mg der (S)-4-(2-Brombenzoyl)-5-hydroxy-benzocyclobuten-1-carbonsäure oder eines Salzes, z. B. des Natriumsalzes, davon, können wie folgt hergestellt werden:

Zusammensetzung (für 1000 Lacktabletten):

| | |
|---|---|
| Wirkstoff | 100,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Dichlormethan | q.s. |

Der Wirkstoff, die Lactose und 40 g der Maisstärke werden gemischt. Das Gemisch wird mit einem Kleister, hergestellt aus 15 g Maisstärke und Wasser (unter Erwärmen), befeuchtet und granuliert. Das Granulat wird getrocknet und der Rest der Malsstärke, der Talk und das Calciumstearat werden mit dem Granulat vermischt. Das Gemisch wird zu Tabletten (Gewicht: je 280 mg) verpresst und diese werden mit einer Lösung der Hydroxypropylmethylcellulose und des Schellacks in Dichlormethan lackiert (Endgewicht der Lacktabletten: je 283 mg).

Präparatebeispiel 3: Gelatinesteckkapseln, als Wirkstoff enthaltend je 100 mg der (S)-4-(2-Brombenzoyl)-5-hydroxy-benzocyclobuten-1-carbonsäure oder eines Salzes, z. B. des Natriumsalzes, davon, können folgendermassen hergestellt werden:

Zusammensetzung (für 1000 Kapseln):

| | |
|---|---|
| Wirkstoff | 100,0 g |
| Lactose | 250,0 g |
| mikrokristalline Cellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig gemischt. Dann wird zunächst die Lactose durch ein Sieb mit einer Maschenweite von 0,6 mm und anschliessend die milcrokristalline Cellulose durch ein Sieb mit einer Maschenweite von 0,9 mm hinzugesiebt. Dann werden alle vier Komponenten 10 Minuten innig gemischt. Zuletzt wird das Magnesiumstearat durch ein Sieb mit einer Maschenweite von 0,8 mm hinzugesiebt. Nach weiterem Mischen (3 Minuten) werden je 390 mg der erhaltenen Formulierung in Gelatinesteckkapseln der Grösse 0 abgefüllt.

Präparatebeispiel 4: Eine Injektions oder Infusionslösung, als Wirkstoff enthaltend die (S)-4-(2-Brombenzoyl)-5-hydroxy-benzocyclobuten-1-carbonsäure oder ein Salz, z. B. das Natriumsalz, davon, kann folgendermassen hergestellt werden:

Zusammensetzung (für 1000 Ampullen):

| | |
|---|---|
| Wirkstoff | 5,0 g |
| Natriumchlorid | 22,5 g |
| Phosphatpufferlösung (pH: 7,4) | 300,0 g |
| entmineralisiertes Wasser | ad 2500,0 ml |

Der Wirkstoff und das Natriumchlorid werden in 1000 ml entmineralisiertem Wasser gelöst. Die Lösung wird durch ein Mikrofilter filtriert. Man versetzt das Filtrat mit der Phosphatpufferlösung und füllt das Gemisch mit entmineralisiertem Wasser auf 2500 ml auf. Zur Herstellung von Dosiseinheitsformen werden je 2,5 ml des Gemisches in Glasampullen abgefüllt, die dann je 5 mg Wirkstoff enthalten.

## Patentansprüche

1. Verfahren zur Herstellung von (S)-4-(2-Brombenzoyl)-5-hydroxy-benzocyclobuten-1-carbonsäure der Formel

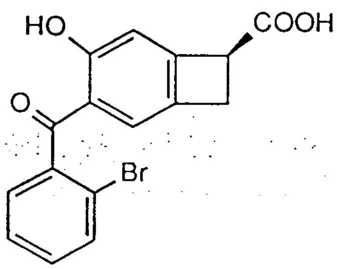

(I),

in freier Form oder in Salzform, dadurch gekennzeichnet, dass man racemische 4-(2-Brombenzoyl)-5-hydroxy-benzocyclobuten-1-carbonsäure in einem alkoholischen Lösungsmittel 6 bis 48 Stunden bei erhöhter Temperatur mit mindestens der äquimolaren Menge Chinin umsetzt, das als unmittelbares Umsetzungsprodukt kristallin ausgefallene Chininsalz der (S)-4-(2-Brombenzoyl)-5-hydroxybenzocyclobuten-1-carbonsäure aus dem Reaktionsgemisch abtrennt, aus diesem Salz durch Säurebehandlung in üblicher Weise die (S)-4-(2-Brombenzoyl)-5-hydroxy-benzocyclobuten-1-carbonsäure freisetzt und diese freie Säure gewünschtenfalls in ein Salz überführt.

2. Verfahren gemäss Anspruch 1, dadurch gekennzeichnet, dass man als alkoholisches Lösungsmittel ein $C_1$-$C_7$-Alkanol verwendet.

3. Verfahren gemäss Anspruch 1 oder 2, dadurch gekennzeichnet, dass man als alkoholisches Lösungsmittel ein $C_1$-$C_4$-Alkanol verwendet.

4. Verfahren gemäss einem der Ansprüche 1 bis 3, dadurch gekennzeichnet, dass man als alkoholisches Lösungsmittel Methanol, Ethanol, Propanol, Isopropanol oder Butanol verwendet.

5. Verfahren gemäss einem der Ansprüche 1 bis 4, dadurch gekennzeichnet, dass man als alkoholisches Lösungsmittel Ethanol verwendet.

6. Verfahren gemäss einem der Ansprüche 1 bis 5, dadurch gekennzeichnet, dass man pro g der racemischen 4-(2-Brombenzoyl)-5-hydroxy-benzocyclobuten-1-carbonsäure 8 bis 50 ml des alkoholischen Lösungsmittels verwendet.

7. Verfahren gemäss einem der Ansprüche 1 bis 6, dadurch gekennzeichnet, dass man pro g der racemischen 4-(2-Brombenzoyl)-5-hydroxy-benzocyclobuten-1-carbonsäure 20 bis 30 ml des alkoholischen Lösungsmittels verwendet.

8. Verfahren gemäss einem der Ansprüche 1 bis 7, dadurch gekennzeichnet, dass man die racemische 4-(2-Brombenzoyl)-5-hydroxy-benzocyclobuten-1-carbonsäure 12 bis 24 Stunden mit Chinin umsetzt.

9. Verfahren gemäss einem der Ansprüche 1 bis 8, dadurch gekennzeichnet, dass man die racemische 4-(2-Brombenzoyl)-5-hydroxy-benzocyclobuten-1-carbonsäure in einem Temperaturbereich von 50° bis 120° C mit Chinin umsetzt.

10. Verfahren gemäss einem der Ansprüche 1 bis 9, dadurch gekennzeichnet, dass man die racemische 4-(2-Brombenzoyl)-5-hydroxy-benzocyclobuten-1-carbonsäure in einem Temperaturbereich von 60° bis 90° C mit Chinin umsetzt.

11. Verfahren gemäss einen, der Ansprüche 1 bis 10, dadurch gekennzeichnet, dass man die racemische 4-(2-Brombenzoyl)-5-hydroxy-benzocyclobuten-1-carbonsäure bei der Siedetemperatur des verwendeten alkoholischen Lösungsmittels mit Chinin umsetzt.

12. Verfahren gemäss einem der Ansprüche 1 bis 11, dadurch gekennzeichnet, dass man die racemische 4-(2-Brombenzoyl)-5-hydroxy-benzocyclobuten-1-carbonsäure mit der 1,0- bis 1,5-fachen molaren Menge Chinin umsetzt.

13. Verfahren gemäss einem der Ansprüche 1 bis 12, dadurch gekennzeichnet, dass man die racemische 4-(2-Brombenzoyl)-5-hydroxy-benzocyclobuten-1-carbonsäure mit der 1,05- bis 1,3-fachen molaren Menge Chinin umsetzt.

14. Verfahren gemäss einem der Ansprüche 1 bis 13, dadurch gekennzeichnet, dass man die racemische 4-(2-Brombenzoyl)-5-hydroxy-benzocyclobuten-1-carbonsäure mit der 1,15- bis 1,25-fachen molaren Menge Chinin umsetzt.

15. Verfahren gemäss einem der Ansprüche 1 bis 14, dadurch gekennzeichnet, dass man das als unmittelbares Umsetzungsprodukt kristallin ausgefallene Chininsalz der (S)-4-(2-Brombenzoyl)-5-hydroxy-benzocyclobuten-1-carbonsäure durch Filtration aus dem Reaktionsgemisch abtrennt.

16. Verfahren gemäss einem der Ansprüche 1 bis 15, dadurch gekennzeichnet, dass man das als unmittelbares Umsetzungsprodukt kristallin ausgefallene Chininsalz der (S)-4-(2-Brombenzoyl)-5-hydroxy-benzocyclobuten- 1-carbonsäure durch Saugfiltration (Abnutschen) aus dem Reaktionsgemisch abtrennt.

17. Verfahren gemäss einem der Ansprüche 1 bis 14, dadurch gekennzeichnet, dass man das als unmittelbares Umsetzungsprodukt kristallin ausgefallene Chininsalz der (S)-4-(2-Brombenzoyl)-5-hydroxy-benzocyclobuten-1-carbonsäure durch Zentrifugation aus dem Reaktionsgemisch abtrennt.

18. Verfahren gemäss einem der Ansprüche 1 bis 17, dadurch gekennzeichnet, dass die Freisetzung der (S)-4-(2-Brombenzoyl)-5-hydroxy-benzocyclobuten-1-carbonsäure aus ihrem verfahrensgemäss erhaltenen Chininsalz durch Behandlung mit einer Mineralsäure erfolgt.

19. Verfahren gemäss einem der Ansprüche 1 bis 18, dadurch gekennzeichnet, dass die Freisetzung der (S)-4-(2-Brombenzoyl)-5-hydroxy-benzocyclobuten-1-carbonsäure aus ihrem verfahrensgemäss erhaltenen Chininsalz durch Behandlung mit einer Halogenwasserstoffsäure erfolgt.

**20.** Verfahren gemäss einem der Ansprüche 1 bis 19, dadurch gekennzeichnet, dass die Freisetzung der (S)-4-(2-Brombenzoyl)-5-hydroxy-benzocyclobuten-1-carbonsäure aus ihrem verfahrensgemäss erhaltenen Chininsalz durch Behandlung mit Salzsäure erfolgt.

**21.** Verfahren gemäss einem der Ansprüche 1 bis 20, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz die zur Freisetzung verwendete Säure mindestens in der äquimolaren Menge eingesetzt wird.

**22.** Verfahren gemäss einem der Ansprüche 1 bis 21, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz die zur Freisetzung verwendete Säure mindestens in der 2- bis 10-fachen molaren Menge eingesetzt wird.

**23.** Verfahren gemäss einem der Ansprüche 1 bis 22, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz die zur Freisetzung verwendete Säure mindestens in der 2- bis 5-fachen molaren Menge eingesetzt wird.

**24.** Verfahren gemäss einem der Ansprüche 1 bis 23, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz in einem Zweiphasensystem gearbeitet wird.

**25.** Verfahren gemäss einem der Ansprüche 1 bis 24, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz in einem Zweiphasensystem, gebildet aus Wasser und einem mit Wasser nichtmischbaren oder nur teilweise mischbaren orginischen Lösungsmittel, gearbeitet wird.

**26.** Verfahren gemäss einem der Ansprüche 1 bis 25, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz in einem Zweiphasensystem, gebildet aus Wasser und einem aliphatischen Alkohol, einem niederen Fettsäureester, einem aromatischen oder araliphatischen Kohlenwasserstoff, einem halogenierten aliphatischen Kohlenwasserstoff oder einem aliphatischen Ether, gearbeitet wird.

**27.** Verfahren gemäss einem der Ansprüche 1 bis 26, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz in einem Zweiphasensystem, gebildet aus Wasser und einem einem $C_4$-$C_7$-Alkanol, einem $C_2$-$C_7$-Alkansäure-$C_1$-$C_4$-alkylester, Benzol oder einem Alkylderivat davon, einem Halogen-$C_1$-$C_4$-alkan oder einem Di-$C_1$-$C_4$-alkylether, gearbeitet wird.

**28.** Verfahren gemäss einem der Ansprüche 1 bis 27, dadurch gekennzeichnet, dass bei der Freisetzung der (S)-4-(2-Brombenzoyl)-5-hydroxy-benzocyclobuten-1-carbonsäure aus ihrem verfahrensgemäss erhaltenen Chininsalz in einem Zweiphasensystem, gebildet aus Wasser und tert.-Butylmethylether, gearbeitet wird.

**29.** Verfahren gemäss Anspruch 1, dadurch gekennzeichnet, dass man eine Lösung von racemischer 4-(2-Brombenzoyl)-5-hydroxy-benzocyclobuten-1-carbonsäure und der 1,0- bis 1,5-fachen molaren Menge Chinin in der 8- bis 50-fachen Volumenmenge eines $C_1$-$C_4$-Alkanols 6 bis 48 Stunden auf 60° bis 90° C erwärmt, das als unmittelbares Umsetzungsprodukt kristallin ausgefallene Chininsalz der (S)-4-(2-Brombenzoyl)-5-hydroxy-benzocyclobuten-1-carbonsäure in üblicher Weise aus dem Reaktionsgemisch abtrennt und die (S)-4-(2-Brombenzoyl)-5-hydroxy-benzocyclobuten-1-carbonsäure, in üblicher Weise durch Säurehehandlung ihres verfahrensgemäss erhaltenen Chininsalzes freisetzt.

**30.** Verfahren gemäss Anspruch 29, dadurch gekennzeichnet, dass man eine Lösung von racemischer 4-(2-Brombenzoyl)-5-hydroxy-benzocyclobuten-1-carbonsäure und der etwa 1,05- bis 1,3-fachen molaren Menge Chinin in der 20- bis 30-fachen Volumenmenge Ethanol 12 bis 24 Stunden auf die Siedetemperatur des Ethanols erwärmt, das als unmittelbares Umsetzungsprodukt kristallin ausgefallene Chininsalz der (S)-4-(2-Brombenzoyl)-5-hydroxy-benzocyclobuten-1-carbonsäure in üblicher Weise aus dem Reaktionsgemisch abtrennt und die (S)-4-(2-Brombenzoyl)-5-hydroxy-benzocyclobuten-1-carbonsäure durch Behandlung ihres verfahrensgemäss erhaltenen Chininsalzes mit der 2- bis 5-fachen molaren Menge Salzsäure in einem Zweiphasensystem, gebildet aus Wasser und tert.-Butylmethylether, freisetzt.

**31.** (S)-4-(2-Brombenzoyl)-5-hydroxy-benzocyclobuten-1-carbonsäure der Formel

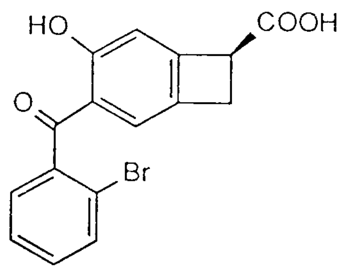

(I),

in freier Form oder in Salzform.

**32.** Die Verbindung gemäss Anspruch 31, in freier Form oder in Form eines pharmazeutisch verwendbaren Salzes, zur Anwendung in einem Verfahren zur therapeutischen Behandlung des menschlichen oder tierischen Körpers.

**33.** Eine Verbindung gemäss Anspruch 31 oder 32, in freier Form oder in Form eines pharmazeutisch verwendbaren Salzes, zur Anwendung als Antinociceptivum, Antiinflammatorikum oder Prostaglandinsynthesehemmer.

**34.** Ein pharmazeutisches Präparat, als Wirkstoff enthaltend eine Verbindung gemäss einem der Ansprüche 31 bis 33, in freier Form oder in Form eines pharmazeutisch verwendbaren Salzes, gegebenenfalls neben üblichen pharmazeutischen Hilfsstoffen.

**35.** Ein antinociceptiv, antiinflammatorisch oder als Prostaglandinsynthesehemmer wirksames pharmazeutisches Präparat gemäss Anspruch 34, dadurch gekennzeichnet, dass man einen antinociceptiv, antiinflammatorisch oder als Prostaglandinsynthesehemmer wirksamen Wirkstoff wählt.

**36.** Verfahren zur Herstellung eines pharmazeutischen Präparats gemäss Anspruch 34 oder 35, dadurch gekennzeichnet, dass man den Wirkstoff, gegebenenfalls unter Beimischung von üblichen pharmazeutischen Hilfsstoffen, zu einem pharmazeutischen Präparat verarbeitet.

**37.** Verfahren gemäss Anspruch 36 zur Herstellung eines antinociceptiv, antiinflammatorisch oder als Prostaglandinsynthesehemmer wirksamen pharmazeutischen Präparats gemäss Anspruch 35, dadurch gekennzeichnet, dass man einen antinociceptiv, antiinflammatorisch oder als Prostaglandinsynthesehemmer wirksamen Wirkstoff wählt.

**38.** Verwendung einer Verbindung gemäss einem der Ansprüche 31 bis 33, in freier Form oder in Form eines pharmazeutisch verwendbaren Salzes, zur Herstellung eines pharmazeutischen Präparats.

**39.** Verwendung einer Verbindung gemäss einem der Ansprüche 31 bis 33, in freier Form oder in Form eines pharmazeutisch verwendbaren Salzes, zur Herstellung eines pharmazeutischen Präparats auf nicht-chemischem Wege.

**40.** Verwendung einer Verbindung gemäss Anspruch 38 oder 39 zur Herstellung eines Antinociceptivums, Antiinflammatorikums oder Prostaglandinsynthesehemmers.

## Claims

**1.** A process for the preparation of (S)-4-(2-bromobenzoyl)-5-hydroxybenzocyclobutene-1-carboxylic acid of the formula

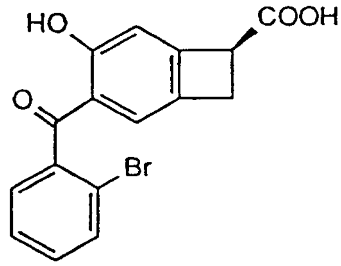

(I),

in free form or in salt form, characterised in that racemic 4-(2-bromobenzoyl)-5-hydroxybenzocyclobutene-1-carboxylic acid is reacted at elevated temperature with at least the equimolar amount of quinine in an alcoholic solvent for 6 to 48 hours, the quinine salt of the (S)-4-(2-bromobenzoyl)-5-hydroxy-benzocyclobutene-1-carboxylic acid, precipitated in crystalline form as the direct reaction product, is separated from the reaction mixture, the (S)-4-(2-bromobenzoyl)-5-hydroxybenzocyclobutene-1-carboxylic acid is set free from this salt by acid treatment in a customary manner, and, if desired, this free acid is converted into a salt.

**2.** A process according to claim 1, characterised in that a $C_1$-$C_7$alkanol is used as the alcoholic solvent.

**3.** A process according to claim 1 or 2, characterised in that a $C_1$-$C_4$alkanol is used as the alcoholic solvent.

**4.** A process according to any one of claims 1 to 3, characterised in that a methanol, ethanol, propanol, isopropanol or butanol is used as the alcoholic solvent.

**5.** A process according to any one of claims 1 to 4, characterised in that ethanol is used as the alcoholic solvent.

**6.** A process according to any one of claims 1 to 5, characterised in that 8 to 50 ml of the alcoholic solvent are used per g of racemic 4-(2-bromobenzoyl)-5-hydroxy-benzocyclobutene-1-carboxylic acid.

**7.** A process according to any one of claims 1 to 6, characterised in that 20 to 30 ml of the alcoholic solvent are used per g of racemic 4-(2-bromobenzoyl)-5-hydroxy-benzocyclobutene-1-carboxylic acid.

**8.** A process according to any one of claims 1 to 7, characterised in that the racemic 4-(2-bromobenzoyl)-5-hydroxy-benzocyclobutene-1-carboxylic acid is reacted with quinine for 12 to 24 hours.

**9.** A process according to any one of claims 1 to 8, characterised in that the racemic 4-(2-bromobenzoyl)-5-hydroxy-benzocyclobutene-1-carboxylic acid is reacted with quinine in a temperature range from 50° to 120°C.

**10.** A process according to any one of claims 1 to 9, characterised in that the racemic 4-(2-bromobenzoyl)-5-hydroxy-benzocyclobutene-1-carboxylic acid is reacted with quinine in a temperature range from 60° to 90°C.

**11.** A process according to any one of claims 1 to 10, characterised in that the racemic 4-(2-bromobenzoyl)-5-hydroxy-benzocyclobutene-1-carboxylic acid is reacted with quinine at the boiling temperature of the alcoholic solvent used.

**12.** A process according to any one of claims 1 to 11, characterised in that the racemic 4-(2-bromobenzoyl)-5-hydroxybenzocyclobutene-1-carboxylic acid is reacted with 1.0 to 1.5 times the molar amount of quinine.

**13.** A process according to any one of claims 1 to 12, characterised in that the racemic 4-(2-bromobenzoyl)-5-hydroxybenzocyclobutene-1-carboxylic acid is reacted with 1.05 to 1.3 times the molar amount of quinine.

**14.** A process according to any one of claims 1 to 13, characterised in that the racemic 4-(2-bromobenzoyl)-5-hydroxy-benzocyclobutene-1-carboxylic acid is reacted with 1.15 to 1.25 times the molar amount of quinine.

**15.** A process according to any one of claims 1 to 14, characterised in that the separation of the quinine salt of (S)-4-(2-bromobenzoyl)-5-hydroxy-benzocyclobutene-1-carboxylic acid, precipitated in crystalline form as the direct reaction product, from the reaction mixture is carried out by filtration.

**16.** A process according to any one of claims 1 to 15, characterised in that the separation of the quinine salt of (S)-4-(2-bromobenzoyl)-5-hydroxy-benzocyclobutene-1-carboxylic acid, precipitated in crystalline form as the direct reaction product, from the reaction mixture is carried out by suction filtration

**17.** A process according to any one of claims 1 to 14, characterised in that the separation of the quinine salt of (S)-4-(2-bromobenzoyl)-5-hydroxy-benzocyclobutene-1-carboxylic acid, precipitated in crystalline form as the direct reaction product, from the reaction mixture is carried out by centrifugation.

**18.** A process according to any one of claims 1 to 17, characterised in that the liberation of the (S)-4-(2-bromobenzoyl)-5-hydroxybenzocyclobutene-1-carboxylic acid from its quinine salt obtained according to the process is carried out by treatment with a mineral acid.

**19.** A process according to any one of claims 1 to 18, characterised in that the liberation of the (S)-4-(2-bromobenzoyl)-5-hydroxybenzocyclobutene-1-carboxylic acid from its quinine salt obtained according to the process is carried out by treatment with a hydrohalic acid.

**20.** A process according to any one of claims 1 to 19, characterised in that the liberation of the (S)-4-(2-bromobenzoyl)-5-hydroxybenzocyclobutene-1-carboxylic acid from its quinine salt obtained according to the process is carried out by treatment with hydrochloric acid.

**21.** A process according to any one of claims 1 to 20, characterised in that in the liberation of the (S)-4-(2-bromobenzoyl)-5-hydroxybenzocyclobutene-1-carboxylic acid from its quinine salt obtained according to the process the acid employed for the liberation is used at least in the equimolar amount.

**22.** A process according to any one of claims 1 to 21, characterised in that in the liberation of the (S)-4-(2-bromobenzoyl)-5-hydroxybenzocyclobutene-1-carboxylic acid from its quinine salt obtained according to the process the acid employed for the liberation is used at least in 2 to 10 times the molar amount.

**23.** A process according to any one of claims 1 to 22, characterised in that in the liberation of the (S)-4-(2-bromobenzoyl)-5-hydroxybenzocyclobutene-1-carboxylic acid from its quinine salt obtained according to the process the acid employed for the liberation is used at least in 2 to 5 times the molar amount.

**24.** A process according to any one of claims 1 to 23, characterised in that the liberation of the (S)-4-(2-bromobenzoyl)-5-hydroxybenzocyclobutene-1-carboxylic acid from its quinine salt obtained according to the process is carried out in a two-phase system.

**25.** A process according to any one of claims 1 to 24, characterised in that the liberation of the (S)-4-(2-bromobenzoyl)-5-hydroxybenzocyclobutene-1-carboxylic acid from its quinine salt obtained according to the process is carried out in a two-phase system, formed from water and a water-immiscible or only partially water-miscible organic solvent.

**26.** A process according to any one of claims 1 to 25, characterised in that the liberation of the (S)-4-(2-bromobenzoyl)-5-hydroxybenzocyclobutene-1-carboxylic acid from its quinine salt obtained according to the process is carried out in a two-phase system, formed from water and an aliphatic alcohol, a lower fatty acid ester, an aromatic or araliphatic hydrocarbon, a halogenated aliphatic hydrocarbon or an aliphatic ether.

**27.** A process according to any one of claims 1 to 26, characterised in that the liberation of the (S)-4-(2-bromobenzoyl)-5-hydroxybenzocyclobutene-1-carboxylic acid from its quinine salt obtained according to the process is carried out in a two-phase system, formed from water and a $C_4$-$C_7$ alkanol, a $C_2$-

$C_7$alkanoic acid-$C_1$-$C_4$alkyl ester, benzene or an alkyl derivative thereof, a halo-$C_1$-$C_4$alkane or a di-$C_1$-$C_4$alkyl ether.

**28.** A process according to any one of claims 1 to 27, characterised in that the liberation of the (S)-4-(2-bromobenzoyl)-5-hydroxybenzocyclobutene-1-carboxylic acid from its quinine salt obtained according to the process is carried out in a two-phase system, formed from water and tert-butyl methyl ether.

**29.** A process according to claim 1, characterised in that a solution of racemic 4-(2-bromobenzoyl)-5-hydroxy-benzocyclobutene-1-carboxylic acid and 1.0 to 1.5 times the molar amount of quinine in 8 to 50 times the amount by volume of a $C_1$-$C_4$alkanol is heated to from 60° to 90°C for 6 to 48 hours, the quinine salt of (S)-4-(2-bromobenzoyl)-5-hydroxybenzocyclobutene-1-carboxylic acid, precipitated in crystalline form as the direct reaction product, is separated from the reaction mixture in a customary manner, and the (S)-4-(2-bromobenzoyl)-5-hydroxy-benzocyclobutene-1-carboxylic acid is liberated in a customary manner by acid treatment of its quinine salt obtained according to the process.

**30.** A process according to claim 29, characterised in that a solution of racemic 4-(2-bromobenzoyl)-5-hydroxy-benzocyclobutene-1-carboxylic acid and 1.05 to 1.3 times the molar amount of quinine in 20 to 30 times the amount by volume of ethanol is heated to the boiling temperature of the ethanol for 12 to 24 hours, the quinine salt of (S)-4-(2-bromobenzoyl)-5-hydroxy-benzocyclobutene-1-carboxylic acid, precipitated in crystalline form as the direct reaction product, is separated from the reaction mixture in a customary manner, and the (S)-4-(2-bromobenzoyl)-5-hydroxy-benzocyclobutene-1-carboxylic acid is liberated by treatment of its quinine salt obtained according to the process with 2 to 5 times the molar amount of hydrochloric acid in a two-phase system formed from water and tert-butyl methyl ether.

**31.** (S)-4-(2-Bromobenzoyl)-5-hydroxy-benzocyclobutene-1-carboxylic acid of the formula

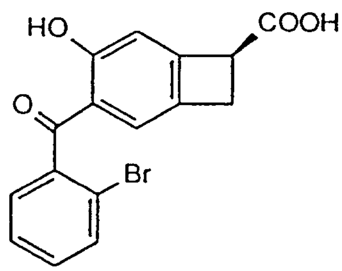

(I),

in free form or in salt form.

**32.** The compound according to claim 31, in free form or in the form of a pharmaceutically acceptable salt, for use in a method for the therapeutic treatment of the human or animal body.

**33.** The compound according to claim 31 or 32, in free form or in the form of a pharmaceutically acceptable salt, for use as an antinociceptive, antiinflammatory or prostaglandin synthesis inhibitor.

**34.** A pharmaceutical preparation containing, as the active ingredient, a compound according to any one of claims 31 to 33, in free form or in the form of a pharmaceutically acceptable salt, if appropriate in addition to customary pharmaceutical adjuncts.

**35.** An antinociceptive, antiinflammatory or prostaglandin synthesis inhibitor according to claim 34, characterised in that an active ingredient having antinociceptive, antiinflammatory or prostaglandin synthesis inhibitor activity is chosen.

**36.** A process for the preparation of a pharmaceutical preparation according to claim 34 or 35, characterised in that the active ingredient is processed to give a pharmaceutical preparation, customary pharmaceutical adjuncts being mixed in if appropriate.

**37.** The process according to claim 36 for the preparation of a pharmaceutical preparation or activity having antinociceptive, antiinflammatory or prostaglandin synthesis inhibitors according to claim 35, characterised in that an active ingredient or activity having antinociceptive, antiinflammatory or prostaglandin synthesis inhibitor activity is chosen.

**38.** The use of a compound according to any one of claims 31 to 33, in free form or in the form of a pharmaceutically acceptable salt, for the preparation of a pharmaceutical preparation.

**39.** The use of a compound according to any one of claims 31 to 33, in free form or in the form of a pharmaceutically acceptable salt, for the preparation of a pharmaceutical preparation by a non-chemical route.

**40.** The use of a compound according to claim 38 or 39 for the preparation of an antinociceptive, antiinflammatory or prostaglandin synthesis inhibitor.

**Revendications**

**1.** Procédé de préparation d'acide (S)-4-(2-bromobenzoyl)-5-hydroxy-benzocyclobutène-1-carboxylique de formule

(I)

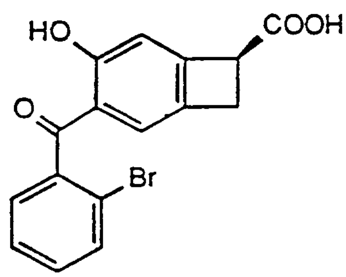

sous forme d'acide libre ou sous forme de sel, caractérisé en ce que l'on fait réagir un mélange racémique d'acide 4-(2-bromobenzoyl)-5-hydroxybenzocyclobutène-1-carboxylique avec une quantité au moins équimolaire de quinine pendant 6 à 48 heures à température élevée dans un solvant alcoolique, en ce que l'on sépare du mélange réactionnel le produit réactionnel direct, c'est-à-dire le (S)-4-(2-bromobenzoyl)-5-hydroxybenzocyclobutène-1-carboxylate de quinine formant un précipité cristallin et en ce que l'on libère à partir de ce sel de manière habituelle par un traitement acide l'acide (S)-4-(2-bromobenzoyl)-5-hydroxybenzocyclobutène-1-carboxylique, cet acide libre pouvant être converti éventuellement en sel.

**2.** Procédé conforme à la revendication 1, caractérisé en ce que l'on utilise comme solvant alcoolique un alcanol en $C_{1-7}$.

**3.** Procédé conforme à la revendication 1 ou 2, caractérisé en ce que l'on utilise comme solvant alcoolique un alcanol en $C_{1-4}$.

**4.** Procédé conforme à une des revendications 1 à 3, caractérisé en ce que l'on utilise comme solvant alcoolique du méthanol, de l'éthanol, de l'isopropanol ou du butanol.

**5.** Procédé conforme à une des revendications 1 à 4, caractérisé en ce que l'on utilise comme solvant alcoolique de l'éthanol.

**6.** Procédé conforme à une des revendications 1 à 5, caractérisé en ce que l'on utilise de 8 à 50 ml de solvant alcoolique par g de mélange racémique d'acide 4-(2-bromobenzoyl)-5-hydroxybenzocyclobutène-1-carboxylique.

**7.** Procédé conforme à une des revendications 1 à 6, caractérisé en ce que l'on utilise de 20 à 30 ml de solvant alcoolique par g de mélange racémique d'acide 4-(2-bromobenzoyl)-5-hydroxybenzocyclobutène-1-carboxylique.

**8.** Procédé conforme à une des revendication 1 à 7, caractérisé en ce que l'on fait réagir l'acide 4-(2-bromobenzoyl)-5-hydroxybenzocyclobutène-1-carboxylique pendant 12 à 24 heures avec de la quinine.

**9.** Procédé conforme à une des revendications 1 à 8, caractérisé en ce que l'on fait réagir le mélange racémique d'acide 4-(2-bromobenzoyl)-5-hydroxybenzocyclobutène-1-carboxylique avec de la quiuine à une température comprise entre 50 et 120 °C.

**10.** Procédé conforme à une des revendications 1 à 9, caractérisé en ce que l'on fait réagir le mélange racémique d'acide 4-(2-bromobenzoyl)-5-hydroxybenzocyclobutène-1-carboxylique avec de la quinine à une température comprise entre 60 et 90 °C.

**11.** Procédé conforme à une des revendication 1 à 10, caractérisé en ce que l'on fait réagir le mélange racémique d'acide 4-(2-bromobenzoyl)-5-hydroxybenzocyclobutène-1-carboxylique avec de la quinine à la température d'ébullition du solvant alcoolique utilisé.

**12.** Procédé conforme à une des revendication 1 à 11, caractérisé en ce que l'on fait réagir le mélange racémique d'acide 4-(2-bromobenzoyl)-5-hydroxybenzocyclobutène-1-carboxylique avec une quantité 1,0 à 1,5 fois molaire de quinine.

**13.** Procédé conforme à une des revendication 1 à 12, caractérisé en ce que l'on fait réagir le mélange racémique d'acide 4-(2-bromobenzoyl)-5-hydroxybenzocyclobutène-1-carboxylique avec une quantité 1,05 à 1,3 fois molaire de quinine.

**14.** Procédé conforme à une des revendication 1 à 13, caractérisé en ce que l'on fait réagir le mélange racémique d'acide 4-(2-bromobenzoyl)-5-hydroxybenzocyclobutène-1-carboxylique avec une quantité 1,15 à 1,25 fois molaire de quinine.

**15.** Procédé conforme à une des revendication 1 à 14, caractérisé en ce que l'on sépare par filtration le précipité cristallin correspondant au produit réactionnel direct, c'est-à-dire au 4-(2-bromobenzoyl)-5-hydroxybenzocyclobutène-1-carboxylate de quinine formé comme produit réactionnel direct du mélange réactionnel.

**16.** Procédé conforme à une des revendication 1 à 14, caractérisé en ce que l'on sépare par filtration sous aspiration le précipité cristallin correspondant au produit réactionnel direct, c'est-à-dire au 4-(2-bromobenzoyl)-5-hydroxybenzocyclobutène-1-carboxylate de quinine formé comme produit réactionnel direct du mélange réactionnel.

**17.** Procédé conforme à une des revendication 1 à 14, caractérisé en ce que l'on sépare par centrifugation le précipité cristallin correspondant au produit réactionnel direct, c'est-à-dire au 4-(2-bromobenzoyl)-5-hydroxybenzocyclobutène-1-carboxylate de quinine formé comme produit réactionnel direct du mélange réactionnel.

**18.** Procédé conforme à une des revendications 1 à 17, caractérisé en ce que la libération de l'acide (S)-4-(2-bromobenzoyl)-5-hydroxybenzocyclobutène-1-carboxylique à partir du sel de quinine obtenu selon le présent procédé se fait par traitement avec un acide minéral.

**19.** Procédé conforme à une des revendications 1 à 18, caractérisé en ce que la libération de l'acide (S)-4-(2-bromobenzoyl)-5-hydroxybenzocyclobutène-1-carboxylique à partir du sel de quinine obtenu selon le présent procédé se fait par traitement avec un acide halogénhydrique.

**20.** Procédé conforme à une des revendications 1 à 19, caractérisé en ce que la libération de l'acide (S)-4-(2-bromobenzoyl)-5-hydroxybenzocyclobutène-1-carboxylique à partir du sel de quinine obtenu selon le présent procédé se fait par traitement avec de l'acide chlorhydrique.

**21.** Procédé conforme à une des revendications 1 à 20, caractérisé en ce que l'on utilise pour la libération de l'acide (S)-4-(2-bromobenzoyl)-5-hydroxybenzocyclobutène-1-carboxylique à partir du sel de quinine obtenu selon le présent procédé une quantité au moins équimolaire de l'acide utilisé.

**22.** Procédé conforme à une des revendications 1 à 21, caractérisé en ce que l'on utilise pour la libération de l'acide (S)-4-(2-bromobenzoyl)-5-hydroxybenzocyclobutène-1-carboxylique à partir du sel de quinine obtenu selon le présent procédé une quantité au moins 2 à 10 fois molaire de l'acide utilisé.

**23.** Procédé conforme à une des revendications 1 à 22, caractérisé en ce que l'on utilise pour la libération de l'acide (S)-4-(2-bromobenzoyl)-5-hydroxybenzocyclobutène-1-carboxylique à partir du sel de quinine obtenu selon le présent procédé une quantité au moins 2 à 5 fois molaire de l'acide utilisé.

**24.** Procédé conforme à une des revendications 1 à 23, caractérisé en ce que la libération de l'acide (S)-4-(2-bromobenzoyl)-5-hydroxybenzocyclobutène-1-carboxylique à partir de son sel de quinine obtenu selon le présent procédé se fait dans un système à deux phases.

**25.** Procédé conforme à une des revendications 1 à 24, caractérisé en ce que la libération de l'acide (S)-4-(2-bromobenzoyl)-5-hydroxybenzocyclobutène-1-carboxylique à partir de son sel de quinine obtenu selon le présent procédé se fait dans un système à deux phases formé d'eau et d'un solvant organique non miscible ou seulement partiellement miscible avec l'eau.

**26.** Procédé conforme à une des revendications 1 à 25, caractérisé en ce que la libération de l'acide (S)-4-(2-bromobenzoyl)-5-hydroxybenzocyclobutène-1-carboxylique à partir de son sel de quinine obtenu selon le présent procédé se fait dans un système à deux phases formé d'eau et d'un alcool aliphatique, d'un ester d'acide gras inférieur, d'un hydrocarbure aromatique ou araliphatique, d'un hydrocarbure aliphatique halogéné ou d'un éther aliphatique.

**27.** Procédé conforme à une des revendication 1 à 26, caractérisé en ce que la libération de l'acide (S)-4-(2-bromobenzoyl)-5-hydroxybenzocyclobutène-1-carboxylique à partir de son sel de quinine obtenu selon le présent procédé se fait dans un système à deux phases formé d'eau et d'un alcanol en $C_{4-7}$, d'un alcanoate en $C_{2-7}$ d'alkyle en $C_{1-4}$, de benzène ou d'un dérivé alkylé de benzène, d'un alcane en $C_{1-4}$ halogéné ou d'un di(alkyle en $C_{1-4}$)-éther.

**28.** Procédé conforme à une des revendication 1 à 27, caractérisé en ce que la libération de l'acide (S)-4-(2-bromobenzoyl)-5-hydroxybenzocyclobutène-1-carboxylique à partir de son sel de quinine obtenu selon le présent procédé se fait dans un système à deux phases formé d'eau et de ter-butylméthyléther.

**29.** Procédé conforme à la revendication 1, caractérisé en ce que l'on chauffe une solution contenant le mélange racémique d'acide 4-(2-bromobenzoyl)-5-hydroxybenzocyclobutène-1-carboxylique et une quantité 1,0 à 1,5 fois molaire de quinine dans 8 à 50 volumes d'un alcanol en $C_{1-4}$ pendant 6 à 48 heures à une température comprise entre 60 et 90 °C, en ce que l'on sépare le précipité cristallin correspondant au produit réactionnel direct, c'est-à-dire au (S)-4-(2-bromobenzoyl)-5-hydroxybenzocyclobutène-1-carboxylate de quinine de manière habituelle du mélange réactionnel et en ce que l'on procède à la libération de l'acide 4-(2-bromobenzoyl)-5-hydroxybenzocyclobutène-1-carboxylique de manière habituelle par traitement acide de son sel de quinine obtenu selon le présent procédé.

**30.** Procédé conforme à la revendication 29, caractérisé en ce que l'on chauffe une solution contenant le mélange racémique d'acide 4-(2-bromobenzoyl)-5-hydroxybenzocyclobutène-1-carboxylique et une quantité 1,05 à 1,3 fois molaire de quinine dans 20 à 30 volumes d'éthanol pendant 12 à 24 heures à la température d'ébullition de l'éthanol, en ce que l'on sépare le précipité cristallin correspondant au produit réactionnel direct, c'est-à-dire au (S)-4-(2-bromobenzoyl)-5-hydroxybenzocyclobutène-1-carboxylate de quinine de manière habituelle du mélange réactionnel et en ce que l'on procède à la libération de l'acide (S)-4-(2-bromobenzoyl)-5-hydroxybenzocyclobutène-1-carboxylique par traitement de son sel de quinine obtenu selon le présent procédé avec une quantité 2 à 5 fois molaire d'acide chlorhydrique dans un système à deux phases formé d'eau et de ter-butylméthyléther.

**31.** Acide (S)-4-(2-bromobenzoyl)-5-hydroxybenzocyclobutène-1-carboxylique de formule

(I)

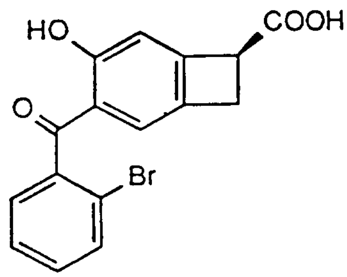

sous forme d'acide libre ou sous forme de sel.

**32.** Composé conforme à la revendication 31, sous forme d'acide libre ou sous forme d'un sel pharmaceutiquement acceptable, pour une application dans un procédé de traitement thérapeutique du corps humain ou animal.

**33.** Composé conforme à la revendication 31 ou 32, sous forme d'acide libre ou de sel pharmaceutiquement acceptable pour une utilisation comme médicament anti-nociceptif, anti-inflammatoire ou inhibiteur de la synthèse des prostaglandines.

**34.** Préparation pharmaceutique contenant, comme principe actif, un composé conforme à une des revendications 31 à 33 sous forme d'acide libre ou sous forme d'un sel pharmaceutiquement acceptable, éventuellement associé à des adjuvants pharmaceutiques habituels.

**35.** Préparation pharmaceutique ayant une action anti-nociceptive, anti-inflammatoire ou inhibitrice de la synthèse des prostaglandines conforme à la revendication 34, caractérisée en ce que l'on choisit un principe actif ayant une action anti-nociceptive, anti-inflammatoire ou inhibitrice de la synthèse des prostaglandines.

**36.** Procédé pour la fabrication d'une préparation pharmaceutique conforme à la revendication 34 ou 35, caractérisé en ce que l'on transforme le principe actif, en y ajoutant éventuellement des adjuvants pharmaceutiques habituels, en une préparation pharmaceutique.

**37.** Procédé conforme à la revendication 36 pour la fabrication d'une préparation pharmaceutique ayant une action anti-nociceptive, anti-inflammatoire ou inhibitrice de la synthèse des prostaglandines conforme à la revendication 35, caractérisé en ce que l'on choisit un principe actif ayant une action anti-nociceptive, anti-inflammatoire ou inhibitrice de la synthèse des prostaglandines.

**38.** Utilisation d'un composé conforme à une des revendications 31 à 33, sous forme d'acide libre ou sous forme d'un sel pharmaceutiquement acceptable, pour la fabrication d'une préparation pharmaceutique.

**39.** Utilisation d'un composé conforme à une des revendication 31 à 33 sous forme d'acide libre ou sous forme d'un sel pharmaceutiquement acceptable, pour la fabrication d'une préparation pharmaceutique selon une voie non chimique.

**40.** Utilisation d'un composé conforme à la revendication 38 ou 39 pour la préparation d'un médicament anti-nociceptif, anti-inflammatoire ou inhibiteur de la synthèse des prostaglandines.